# EUROPÄISCHE PATENTANMELDUNG

(11) **EP 1 333 098 A2**
(43) Veröffentlichungstag der Anmeldung: **06.08.2003**
(21) Anmeldenummer: 03005101.5
(22) Anmeldetag: 24.06.1999
(51) Int. Cl.: C12Q 1/26, G01N 33/50, A01N 43/78

(54) **Verfahren zur Identifizierung von Hemmstoffen der Ferredoxin: NADP Reduktase**

(30) Priorität: 26.06.1998 DE 19828509
(62) Teilanmeldung aus: 99112141.9
(71) Anmelder: BASF AKTIENGESELLSCHAFT, 67056 Ludwigshafen (DE)
(72) Erfinder: Wagner, Oliver, Dr., 67433 Neustadt (DE); Röhl, Franz, Dr., 67105 Schifferstadt (DE); Grossmann, Klaus, Prof.Dr., 67141 Neuhofen (DE); Schmidt, Ralf-Michael, Dr., 67489 Kirrweiler (DE); Sonnewald, Uwe, Prof.Dr., 06484 Quedlinburg (DE); Hajirezaei, Mohammad, Dr., 06466 Gatersleben (DE)

(57) **Zusammenfassung**

Es werden Hemmstoffe der Ferredoxin:NADP Reduktase, ein Testsystem zur Suche nach solchen Hemmstoffen sowie deren Verwendung als Herbizide beschrieben.

## Beschreibung

Die vorliegende Erfindung betrifft Hemmstoffe der Ferredoxin:NADP Reduktase, ein Testsystem zur Suche nach solchen Hemmstoffen sowie deren Verwendung als Herbizide.

Ferredoxin:NADP Reduktase (EC 1.18.1.2; FNR) ist ein Enzym, welches bei der Bereitstellung von Reduktionsäquivalenten für die Assimilation von Kohlenstoff, Stickstoff und Schwefel in Pflanzen beteiligt ist. Das Enzym katalysiert die folgende Reaktion:

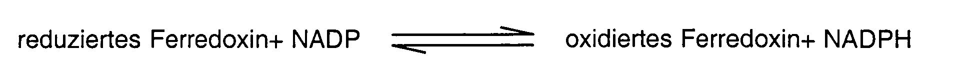

Die Reaktion läuft bei der Photosynthese unter Bildung von NADPH ab (Shin, M. und Arnold, D.I., J. Biol. Chem. 240(1965), 1405-1411) während in nicht-photosynthetischem Gewebe die Bildung von reduziertem Ferredoxin erfolgt (Bowsher, C.G. et al., Plant J. 3(1993), 463-467).

Es ist bekannt, daß Hemmstoffe der Photosynthese zur Verwendung als Herbizide geeignet sind. Diese Herbizide wirken wie z. B. Atrazin als Hemmstoffe des Photosystems II (Hock, B., Fedtke, C. und Schmidt, R.R. (1995) Herbizide, Georg Thieme Verlag) oder wie z. B. Paraquat als Redoxkatalysatoren am Photosystem I (Hock, B., Fedtke, C. und Schmidt, R.R. (1995) Herbizide, Georg Thieme Verlag).

Es wurde berichtet, daß das Herbizid Oxyfluorfen bei Belichtung eine Wirkung auf die Ferredoxin:NADP Reduktase ausübt (Gillham et al. (1985) Proc. Br. Crop Prot. Conf. - Weeds, 1195-1201). Angesichts der bekannten Wirkung von Oxyfluorfen auf die Protoporphyrinogen-IX-Oxidase (Hock, B., Fedtke, C. und Schmidt, R.R. (1995) Herbizide, Georg Thieme Verlag) und die damit verbundene lichtabhängige Bildung von reaktivem Sauerstoff ist diese Wirkung jedoch auf indirekte Effekte zurückzuführen und daher nicht für die herbizide Wirkung von Bedeutung.

Hemmstoffe der Ferredoxin:NADP Reduktase sind als Herbizide bisher nicht bekannt. Es ist anzunehmen, daß die Hemmung der Bereitstellung von Reduktionsäquivalenten für die Assimilation das Wachstum von Pflanzen stark beeinflußt.

Aufgabe der vorliegenden Erfindung war es, Hemmstoffe des Enzyms der Ferredoxin:NADP Reduktase zur Verfügung zu stellen und deren Verwendung als Herbizide zu finden.

Diese Aufgabe wird gelöst durch Hemmstoffe der Ferredoxin:NADP Reduktase der Formel I, wobei =N 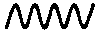 NH- für die E/Z-Isomeren steht, die unter Verwendung eines Ferredoxin:NADP Reduktase Tests erstmals gefunden wurden. 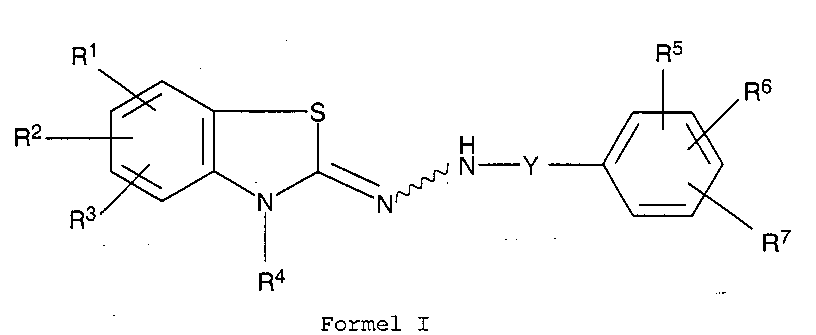
- R¹, R², R³:: jeweils unabhängig voneinander Wasserstoff, Halogen, Cyano, C₁-C₆-Alkyl, C₁-C₆-Alkoxy, C₁-C₆-Alkoxyalkyl, C₁-C₆-Halogenalkyl, C₁-C₆-Alkylthio, C₁-C₆-Halogenalkoxy, C₁-C₆-Alkylcarbonyl, C₁-C₆-Alkoxycarbonyl, S (O)ₙ-C₁-C₆-Alkyl, S(O)ₙ-C₁-C₆-Halogenalkyl, S(O)ₙ-N-C₁-C₆-Alkyl, S(O)ₙ-N(C₁-C₆-Alkyl)₂ mit n = 0, 1, 2, SO₃H, COOH, PO₃H, CONH₂, CONH-C₁-C₆-Alkyl, CO-N(C₁-C₆-Alkyl)₂, NH₂, NH-C₁-C₆-Alkyl, N-(C₁-C₆-Alkyl)₂,
- R⁴:: Wasserstoff, C₁-C₆-Alkyl,
- Y:: CO, SO₂
- R⁵,R⁶,R⁷:: Wasserstoff, Halogen, Cyano, C₁-C₃₀-Alkyl, C₁-C₃₀-Alkoxyalkyl, C₁-C₃₀-Halogenalkyl, C₁-C₃₀-Alkylthio, C₁-C₃₀-Halogenalkoxy, C₁-C₃₀-Alkylcarbonyl, C₁-C₃₀-Alkoxycarbonyl, SO₃H, S(O)ₙ-C₁-C₃₀-Alkyl, S(O)ₙ-C₁-C₃₀-Halogenalkyl, S(O)ₙ-N-C₁-C₃₀-Alkyl, S(O)ₙ-N(C₁-C₃₀-Alkyl)₂ mit n = 0, 1, 2, COO_{H}, CONH₂, CONH-C₁-C₃₀-Alkyl, CO-N(C₁-C₃₀-Alkyl)₂, NH₂, NH-C₁-C₃₀-Alkyl, N-(C₁-C₃₀-Alkyl)₂

Die Verbindungen der allgemeinen Formel I sind teilweise literaturbekannt oder können in analoger Weise hergestellt werden (z. B. GB 975932, BE 704027, BE 665033, DE 1942661, Lieb. Ann. Chem. 700 (1966) 65). 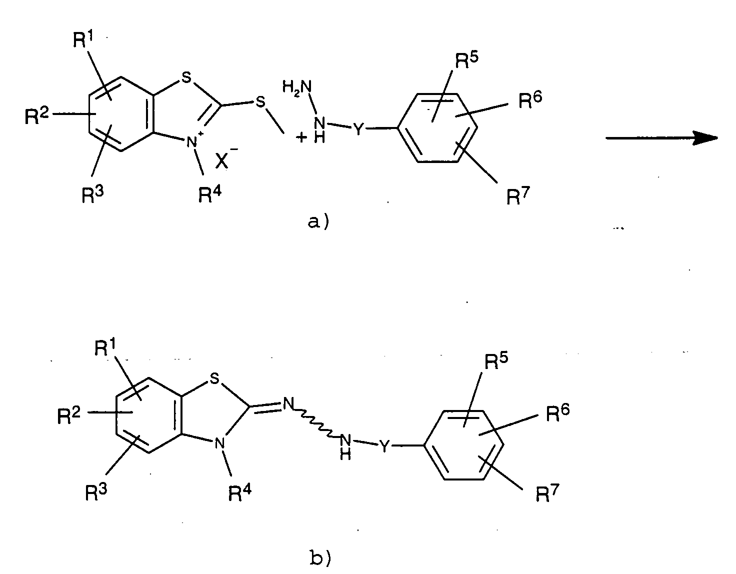 wobei Y = CO oder SO₂ ist und R⁵, R⁶, R⁷ folgende Reste unabhängig voneinander bedeuten können: Wasserstoff, Halogen, Cyano, C₁-C₃₀-Alkyl, C₁-C₃₀-Alkoxyalkyl, C₁-C₃₀-Halogenalkyl, C₁-C₃₀-Alkylthio, C₁-C₃₀-Halogenalkoxy, C₁-C₃₀-Alkylcarbonyl, C₁-C₃₀-Alkoxycarbonyl, SO₃H, S(O)ₙ-C₁-C₃₀-Alkyl, S(O)ₙ-C₁-C₃₀-Halogenalkyl, S(O)ₙ-N-C₁-C₃₀-Alkyl, S(O)ₙ-N(C₁-C₃₀-Alkyl)₂ mit n = 0, 1, 2, COOH, CONH₂, CONH-C₁-C₃₀-Alkyl, CO-N(C₁-C₃₀-Alkyl)₂, NH₂, NH-C₁-C₃₀-Alkyl, N-(C₁-C₃₀-Alkyl)₂,

Alternativ lassen sich Verbindungen der Formel I wie in BE 704027 oder Lieb. Ann. Chem. 623 (1959), 191 beschrieben herstellen: 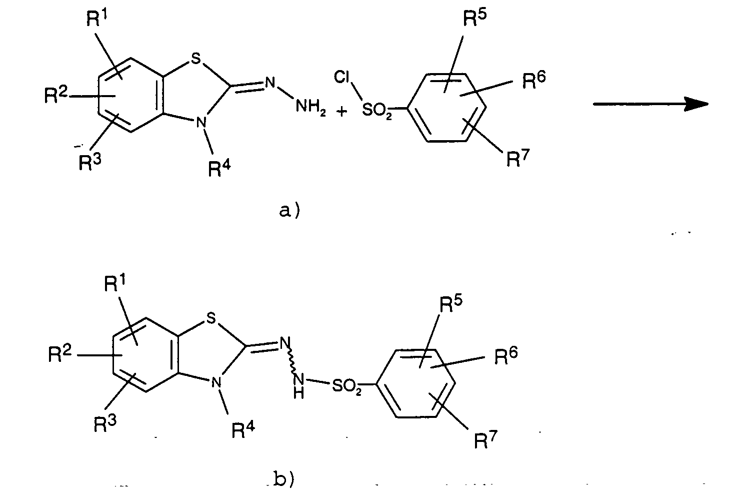 Tabelle A enthält Verbindungen der Formel I A wobei 1 Zeile jeweils einer Verbindung der Formel I A entspricht.

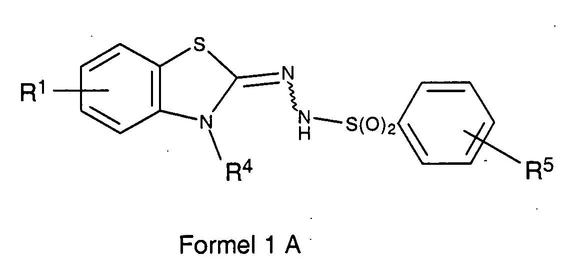

**Tabelle A**

| | R¹ | R⁴ | R⁵ |
|---|---|---|---|
| A.1 | H | Et | 4-NH₂ |
| A.2 | H | Me | 2,5-di-Cl |
| A.3 | H | Me | 4-COOH |
| A.4 | H | Me | 3-SO₃H |
| A.5 | H | Me | 4-Me |
| A.6 | H | Me | H |
| A.7 | H | Me | 4-Me |
| A.8 | H | Me | 3-NO₂ |
| A.9 | H | Me | H |
| A.10 | H | Me | 3-O-(CH₂)₁₁Me |
| A.11 | 6-MeO | Me | H |
| A.12 | H | Me | 2,4-di-C(Me)₂C₂H₅ |
| A.13 | H | Et | H |
| A.14 | H | Me | 2-O-(CH₂)₁₅Me-5-COOH |
| A.15 | 5-F2CHSO2 | Me | 3-COOH-4-SO₂-(CH₂)₁₅Me |
| A.16 | 5-SO3H | Me | 3-COOH-4-SO₂-(CH₂)₁₅Me |
| A.17 | H | Me | 3-COOH-4-O-(CH₂)₁₅Me |
| A.18 | H | Me | 2-O-(CH₂)₁₅Me-5-COOH |
| A.19 | 6-SO3H | Me | 3-COOH-4-O-(CH₂)₁₅Me |
| A.20 | 5-SO3H | Me | 3-COOH-4-O-(CH₂)₁₅Me |
| A.21 | 5-SO2CHF2 | Me | 3-COOH-4-O-(CH2)15Me |
| A.22 | 6-F | Me | 2-O-(CH₂)₁₅Me-5-COOH |
| A.23 | 6-Cl | Me | 2-O-(CH₂)₁₅Me-5-COOH |
| A.24 | 4,6-di-Cl | Me | 2-O-(CH₂)₁₅Me-5-COOH |
| A.25 | 5-CF₃ | Me | 2-O-(CH₂)₁₅Me-5-COOH |
| A.26 | 5-Cl | Me | 2-O-(CH₂)₁₅Me-5-COOH |
| A.27 | H | Me | 2-O-(CH₂)₁₅Me-5-COOEt |
| A.28 | 5-SO₃H | Me | 2-O-(CH₂)₁₅Me-5-COOH |
| A.29 | H | Me | 4-COOEt |
| A.30 | H | Me | 4-OMe |
| A.31 | H | Me | 4-Cl |
| A.32 | H | Me | 2-NO₂ |
| A.33 | H | Et | 3-NH₂ |
| A.34 | 5-SO₂CHF₂ | Me | 3-COOH-4-SO₂-(CH₂)₁₅Me |
| A.35 | 5-SO₃H | Me | 3-COOH-4-SO₂-(CH₂)₁₅Me |
| A.36 | H | Me | 4-NO₂ |
| A.37 | H | Et | 3-NH-CO-C₉H₁₉ |
| A.38 | 6-SO₂NH₂ | Me | 3-COOH-4-O-(CH₂)₁₅Me |
| A.39 | H | Et | 3-NH₂ |
| A.40 | H | CH=CH | 4-NHCOMe |
| A.41 | H | Me | 3-COOH-4-OH |
| A.42 | H | Et | 4-NO₂ |
| A.43 | H | Me | 4-SO₂F |
| A.44 | H | Me | 4-SO₃H |
| A.45 | 6-O-Me | Me | 3-COOH-4-OH |
| A.46 | 6-SO₃H | Me | 3-NHCOMe-4-SO₂-C₁₆H₃₃ |
| A.47 | 5-SO₂CF₂ | Me | 3-NHSO₂-C₁₆H₃₃ |
| A.48 | 5-SO₃H | Me | 3-NMeSO₂-C₁₆H₃₃ |
| A.49 | 5-SO₃H | Me | 3-COOH-4-SO₂-C₁₆H₃₃ |
| A.50 | H | Me | 4-O-C₁₆H₃₃ |
| A.51 | H | Me | 4-SO₂-NH-C₁₆H₃₃ |
| A.52 | 5-SO₃H | Me | 4-SO₂-NH-C₁₆H₃₃ |
| A.53 | H | Me | 4-SO₂-NMe-C₁₆H₃₃ |
| A.54 | C₁₅H₃₁ | Me | 3-COOH |
| A.55 | 5-SO₂CF₂H | Me | 4-SO₂-NH-C₁₆H₃₃ |
| A.56 | 5-SO₂CF₂H | Me | 3-CO-NH-C(C₄H₉)₃ |
| A.57 | H | Me | 4-CO-NHMe |
| A.58 | 5-SO₂CF₂H | Me | 4-SO₂-NH-C(C₄H₉)₃ |
| A.59 | 4,6-di-Cl | Me | 2-O-C₁₆H₃₃-5-COOH |

Tabelle B enthält Verbindungen der Formel I B wobei 1 Zeile jeweils einer Verbindung der Formel I B entspricht.

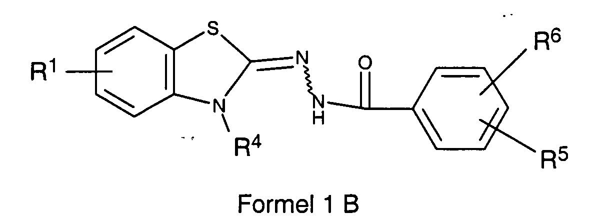

**Tabelle B**

| | R¹ | R⁴ | R⁵ | R⁶ |
|---|---|---|---|---|
| B.1 | H | Et | H | H |
| B.2 | H | Me | H | H |
| B.3 | 5-SO₃H | CH3 | 3-SO₃H | 6-SO₂-C₁₆H₃₃ |

Tabelle C enthält Verbindungen der Formel I C wobei 1 Zeile jeweils einer Verbindung der Formel I C entspricht.

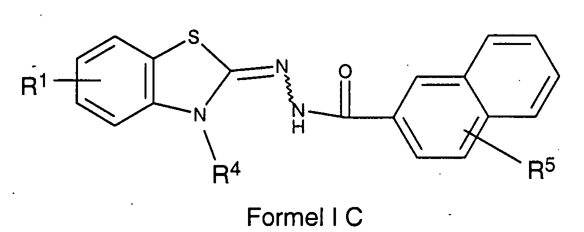

**Tabelle C**

| | R¹ | R⁴ | R⁵ |
|---|---|---|---|
| C.1. | H | Me | 2-OH |

Die Wirkung von Herbiziden kann durch die Herstellung transgener Pflanzen simuliert werden. Solche Pflanzen enthalten die Antisense-Information eines Gens was zur Reprimierung des Genes führt und somit der Situation einer Hemmung des Enzyms entspricht, wie es am Beispiel der Acetolactat-Synthase gezeigt wurde (Höfgen et al., Plant Physiol. 107(1995), 469-477).

Die weiter unten aufgeführten Bei-spiele 1-3 beschreiben die Herstellung einer transgenen Tabakplanze, die das Antisense-Konstrukt zum Gen der Ferredoxin: NADP-Reduktase exprimiert.

Abbildung 1 zeigt die Nukleotid und Protein-Sequenzen des kompletten FNR-Klons.

Abbildung 2 zeigt die Konstruktionszeichnung des chimären Gens, das die Expression von Antisense-RNA der NADP-abhängigen FNR vermittelt.

Demgemäß wurden transgene Pflanzen hergestellt, die eine verminderte Expression der Ferredoxin:NADP Reduktase aufweisen. Wie in Beispiel 4 ersichtlich, ist bei diesen Pflanzen tatsächlich ein reduziertes Wachstum und eine reduzierte FNR-Enzymaktivität zu beobachten.

Die vorliegende Erfindung betrifft weiterhin die Verwendung eines Testsystems zur Suche nach Hemmstoffen der Ferredoxin:NADP Reduktase mit potentiell herbizider Wirkung, sowie Verfahren zur Identifizierung von Stoffen, welche eine potentielle herbizide oder wachstumsregulatorische Wirkung besitzen, die durch eine Hemmung oder Inaktivierung einer planzlichen-Ferredoxin:NADP Reduktase zustande kommt, wobei
a) zunächst das Enzym Ferredoxin:NADP Reduktase durch heterologe Expression einer DNA-Sequenz, die für dieses Transportprotein kodiert, in einem transgenen Organismus oder transgenen Zellen hergestellt wird, anschließend
b) dieser rekombinate Organismus als Ganzes oder ein Zellaufschluß dieses Organismus zur Untersuchung einer chemischen Verbindung auf ihre inhibitorische Wirkung gegen das Enzym Ferredoxin:NADP Reduktase eingesetzt wird und
c) die gegen das Enzym Ferredoxin:NADP Reduktase aktive Verbindung auf ihre herbizide oder wachstumsregulatorische Aktivität gegen Pflanzen geprüft wird.

Als rekombinanter Organismus kann hierbei ein einzelliger Organismus oder eine pflanzliche Zelle eingesetzt werden.

Das Enzym Ferredoxin-NADP-Reduktase (Ferredoxin-NADP Oxidoreduktase, E.C. 1.18.1.2) aus Spinatblättern -erhältlich bei der Fa. Sigma, Deutschland - kann zum Aufbau des Testsystems verwendet werden.
- die Verwendung der Verbindungen der Formel I als Herbizide oder zur Desikkation/Defoliation von Pflanzen,
- herbizide Mittel und Mittel zur Desikkation/Defoliation von Pflanzen, welche die Verbindungen der Formel I als wirksame Substanzen enthalten,
- Verfahren zur Herstellung der Verbindungen der Formel I und von herbiziden Mitteln und Mitteln zur Desikkation/Defoliation von Pflanzen unter Verwendung der Verbindungen der Formel I,
- Verfahren zur Bekämpfung von unerwünschtem Pflanzenwuchs und zur Desikkation/Defoliation von Pflanzen mit den Verbindungen der Formel I,

Ferner wurden herbizide Mittel gefunden, die die Verbindungen der Formel I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen der Formel I gefunden.

Des weiteren wurde gefunden, daß die Verbindungen der Formel I auch zur Desikkation/Defoliation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und Verfahren zur Desikkation und/oder Defoliation von Pflanzen mit den Verbindungen der Formel I gefunden.

Unter landwirtschaftlich brauchbaren Salzen sind vor allem die Salze von I mit denjenigen Kationen sowie Säureadditionssalze von I mit solchen Säuren zu verstehen, welche die herbizide oder desikkante/defoliante Wirkung von I nicht negativ beeinträchtigen.

So kommen als Kationen insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das einen C₁-C₄-Alkyl-, Phenyl- oder Benzylsubstituenten und gewünschtenfalls zusätzlich ein bis drei weitere C₁-C₄-Alkylreste tragen kann, vorzugsweise Diisopropylammonium, Tetramethylammonium, Tetrabutylammonium, Trimethylbenzylammonium, des weiteren Phosphoniumionen, vorzugsweise Tri-(C₁-C₄-alkyl)-phosphonium, Sulfoniumionen, vorzugsweise Tri-(C₁-C₄-alkyl)-sulfonium, sowie Sulfoxoniumionen, vorzugsweise Tri-(C₁-C₄-alkyl)-sulfoxonium, in Betracht.

Landwirtschaftlich brauchbare Salze der Verbindungen der Formel I können durch Reaktion mit einer Base des entsprechenden Kations, vorzugsweise einem Alkalimetallhydroxid oder -hydrid, oder durch Reaktion mit einer Säure des entsprechenden Anions, vorzugsweise der Chlorwasserstoffsäure, Bromwasserstoffsäure, Schwefelsäure, Phosphorsäure oder Salpetersäure, gebildet werden.

Salze von I, deren Metallion kein Alkalimetallion ist, können auch durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen der Formel I und deren landwirtschaftlich brauchbare Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Des weiteren eignen sich die substituierten Verbindungen der Formel I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen der Formel I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die folgenden Formulierungsbeispiele verdeutlichen die Herstellung solcher Zubereitungen:
I. 20 Gewichtsteile der Verbindung der Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile der Verbindung der Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile der Verbindung der Formel I werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile der Verbindung der Formel I werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile der Verbindung der Formel I werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung der Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Anschließend kann mit Wasser auf die gewünschte Wirkstoffkonzentration verdünnt werden. Man erhält ein stabiles Emulsionskonzentrat.
VIII. 1 Gewichtsteil der Verbindung der Formel I wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl; BASF AG) besteht. Danach kann mit Wasser auf die gewünschte Wirkstoffkonzentration verdünnt werden. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindungen der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die substituierten Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxyund Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Beispiel 1

Herstellung einer transgenen Tabakpflanze, die das Antisense-Konstrukt zum Gen der Ferredoxin:NADP Reduktase exprimiert.

### A. Allgemeine Klonierungsverfahren

Klonierungsverfahren wie z.B.: Restriktionsspaltungen, Agarose-Gelelektophorese, Reinigung von DNA-Fragmenten, Transfer von Nukleinsäuren auf Nitrozellulose und Nylon Membranen, Verknüpfen von DNA-Fragmenten, Transformation von *E.coli* Zellen, Anzucht von Bakterien, Vermehrung von Phagen und Sequenzanalyse rekombinanter DNA wurden wie bei Sambrook et al. (1989) Cold Spring Harbor Laboratory Press (ISBN 0-87969-309-6) beschrieben durchgeführt. Die Transformation von *Agrobacterium tumefaciens* wurde entsprechend der Methode von Höfgen und Willmitzer (Nucl. Acid Res. 16 (1988), 9877) ausgeführt. Die Anzucht der Agrobakterien erfolgte in YEB Medium (Vervliet et al., J. Gen. Virol. 26 (1975), 33).

### B. Erzeugung von cDNA-Bibliotheken

Zur Herstellung von Blatt-spezifischen cDNA Bibliotheken wurde Gesamt-RNA aus Blättern von untransformierten Tabakpflanzen (Sonnewald Plant J. 2 (1992), 571) nach einer von Logemann et al. (Anal. Biochem. 163 (1987), 21) beschriebenen Methode isoliert. Anschließend wurde die poly (A) -RNA über Oligo(dT)-Cellulose Type 7 (Pharmacia Biotech Europe, Munzgerstr. 9, 79111 Freiburg) nach Angaben des Herstellers gereinigt. Nach photometrischer Konzentrationsbestimmung wurden 5 µg der so erhaltenen RNA für die cDNA Synthese eingesetzt. Alle für die Herstellung der cDNA notwendigen Chemikalien und Enzyme wurden durch die Firma Stratagene (11099 North Torrey Pines Road, La Jolla, CA 92037, USA) bezogen. Die angewandten Methoden wurden nach Angaben des Herstellers durchgeführt. Die Synthese des ersten und zweiten Stranges der cDNA wurde mit dem λ-ZAP-cDNA Synthese Kit durchgeführt. Die erhaltenen doppelsträngigen cDNAs wurden anschließend mit EcoRI-NotI Adaptoren versehen und in einen EcoRI verdauten Lambda ZAPII Vektor kloniert. Nach in vitro Verpackung (Gigapack II Verpackungsextrakt) der rekombinanten Lambda DNA wurden XL-1 *E.coli* Zellen (Stratagene) transformiert. Durch Auszählen der gebildeten Plaques wurde der Titer der cDNA-Bibliotheken bestimmt.

### C. Erzeugung polyklonaler Antikörper, die gegen membrangebundene Proteine gerichtet sind

Aus ca. 400 g Blattmaterial von Tabakpflanzen (*Nicotiana tabacum* L. cv. Samsun NN) wurden membrangebundene Proteine mittels differenzieller Zentrifugation nach Standardverfahren isoliert. Zur Gewinnung von Antiseren wurden 100 µg Protein für die Immunisierung von Mäusen eingesetzt.

### D. Isolierung von cDNA-Klonen, die für membrangebundene Proteine kodieren

Zur Isolierung von cDNA-Klonen, die für membrangebundene Proteine aus Tabak kodieren, wurde eine blattspezifische cDNA-Bank in λ-ZAP II (Stratagene) mit Hilfe der hergestellten Antiseren durchmustert. Die cDNA-Bank wurde mit einem Titer von 3 x 10⁵ Plaque bildenden Einheiten ausplattiert. Von diesen Platten wurden Plaques auf Nitrocellulose-Filter, die vorher mit 10 mM IPTG für 15 Minuten getränkt wurden, transferiert. Zum Nachweis von Phagen, die membrangebundene Proteine kodierende cDNA-Klone tragen, wurden die Nitrocellulosefilter mit den spezifischen Antikörpern (siehe C) inkubiert. Die Bindung der spezifischen Antikörper wurde anschließend durch einen enzymgekoppelten zweiten Antikörper und darauffolgender Farbreaktion nachgewiesen (Amersham-Buchler). Diese wurden anschließend ausgestochen und einer zweiten Durchmusterung unterzogen, um einheitliche Populationen zu erhalten. 4 Klone wurden bis auf Einzelplaques gereinigt und sequenziert. Der 1050 Basenpaare lange Klon OR15 kodiert für ein 291 langes Polypeptid. Der Homologievergleich ergab, daß dieses Polypeptid zu 80 % identisch mit der FNR aus Bohne ist.

### E. Isolierung eines kompletten FNR-Klones

Da der Klon OR15 nicht die gesamte Kodierungsregion der FNR trägt, wurde zur Isolierung eines kompletten FNR-Klones eine blattspezifische cDNA-Bank in λ-ZAP II (Stratagene) mit einem Titer von 3 x 10⁵ Plaque bildenden Einheiten ausplattiert. Von diesen Platten wurden Plaques auf Nylon-Membran transferiert und diese mit den als Sonde (OR15) verwendeten, radioaktiv markierten cDNA-Fragmenten hybridisiert. Aufgrund der Hybridisierung der Sonde mit homologen cDNA-Fragmenten auf den Plaquefiltern erscheinen durch die Radioaktivität hervorgerufene Schwärzungen auf dem Röntgenfilm. Unter Verwendung dieser Röntgenfilme ist es möglich, die Phagen zu identifizieren, die die gesuchten cDNA-Fragmente tragen. Die isolierten Phagen werden in SM-Medium aufbewahrt. 4 Klone wurden erhalten und durch Restriktionsanalysen charakterisiert. Da sie sich nur in der Sequenzlänge etwas unterscheiden, wird die Sequenz des längsten Klones unter F angegeben.

### F. Sequenzieren der Plasmid-DNA

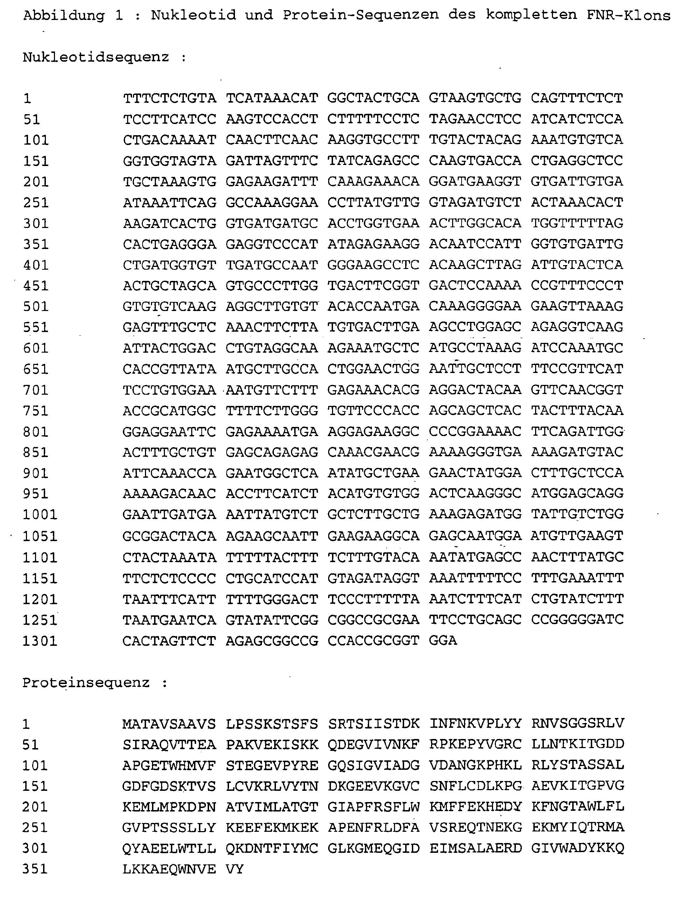

Die im pBluescript enthaltenen cDNA-Klone wurden unter Verwendung spezifischer Primer sequenziert. Die Bindungsstellen dieser Primer sind im pBluescript so gewählt, daß sie Sequenzierungsreaktionen vom 5'-Ende und vom 3'-Ende der cDNA-Klone ermöglichen. Im folgenden sind die Nukleotid- ( publiziert in Jamsen, T. et al Current Genetics 13(1988), 517-522 ) und Protein-Sequenz ( publiziert in Karplus, P.A. et al. Biochemistry 23(1984), 6576-6583 ) der identifizierten FNR gezeigt. Der komplette FNR-Klon ist 1333 Basenpaare lang und trägt einen offenen Leseraster, der für ein 362 Aminosäuren-langes Polypeptid kodiert (Abb.1).

### G. Bakterienstämme

E.coli (XL-1 Blue) Bakterien wurden durch die Firma Stratagene bezogen. Der zur Pflanzentransformation eingesetzte Agrobacterium tumefaciens Stamm (C58C1 mit dem Plasmid pGV 3850kan) wurde von Debleare et al., Nucl. Acid Res. 13(1985), 4777 beschrieben.

### H. Tabaktransformation

Zur Transformation von Tabakpflanzen (*Nicotiana tabacum* L. cv. Samsun NN) wurden 10ml einer unter Selektion gewachsenen Übernachtkultur von *Agrobacterium tumefaciens* abzentrifugiert, der Überstand verworfen, und die Bakterien im gleichen Volumen Antibiotika-freien Mediums resuspendiert. In einer sterilen Petrischale wurden Blattscheiben steriler Pflanzen (Durchmesser ca. 1cm) in dieser Bakterienlösung gebadet. Anschließend wurden die Blattscheiben in Petrischalen auf MS-Medium (Murashige und Skoog, Physiol. Plant. 15 (1962), 473) mit 2 % Saccharose und 0.8 % Bacto-Agar ausgelegt. Nach 2-tägiger Inkubation im Dunkeln bei 25°C wurden sie auf MS-Medium mit 100 mg/l Kanamycin, 500 mg/l Claforan, 1 mg/ Benzylaminopurin (BAP), 0.2 mg/l Naphtylessigsäure (NAA), 1.6 % Glukose und 0.8 % Bacto-Agar übertragen und die Kultivierung (16 Stunden Licht / 8 Stunden Dunkelheit) fortgesetzt. Wachsende Sprosse wurden auf hormonfreies MS-Medium mit 2 % Saccharose, 250 mg/l Claforan und 0.8% Bacto-Agar überführt.

### I. Analyse von Gesamt-RNA aus pflanzlichen Geweben

Gesamt RNA aus pflanzlichen Geweben wurde wie bei Logemann et al. ( Anal. Biochem. 163 (1987), 21) beschrieben isoliert. Für die Analyse wurden jeweils 20-40 µg RNA in einem Formaldehyd-haltigen 1.5 %igen Agarosegel aufgetrennt. Nach elektrophoretischer Auftrennung der RNA Moleküle wurde die RNA mittels Kapillartransfer auf eine Nylon-Membran übertragen. Der Nachweis spezifischer Transkripte wurde wie bei Amasino (Anal. Biochem. 152 (1986), 304) beschrieben durchgeführt. Die als Sonde eingesetzten cDNA-Fragmente wurden mit einem Random Primed DNA Labeling Kit (Boehringer, Mannheim) radioaktiv markiert.

### J. Herstellung des Plasmides pBinAR-FNR-Antisense

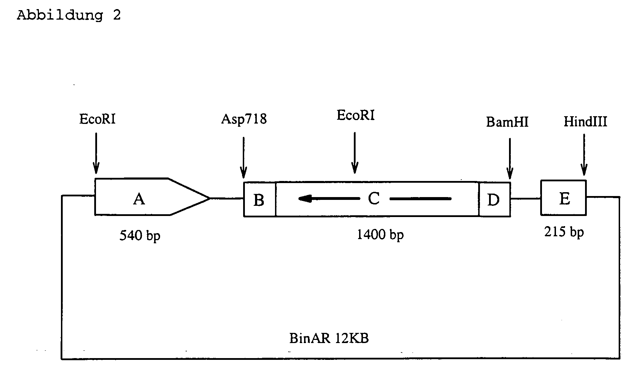

Das Plasmid pBinAR-FNR-Antisense besteht aus den fünf Fragmenten A, B, C, D und E (Abb. 2). Der Expressionsvektor pBinAR enthält das Fragment A, das den 35S CaMV Promoter beinhaltet. Dieses umfaßt ein Fragment, welches die Nukleotide 6909 bis 7437 des Cauliflower-Mosaik Virus (CaMV) umfaßt (Franck et al. Cell 21 (1980), 285). Es wurde als EcoRI-KpnI Fragment aus dem Plasmid pDH51 (Pietrzak et al. Nucleic. Acid Res. 14 (1986), 5857) isoliert. Fragmente B und D enthalten eine Teilsequenz der multiplen Klonierungsstelle und das Fragment C (FNR cDNA) wurde aus dem pBluescript SK als Asp718-BamHI Fragment isoliert und die FNR-cDNA als Asp718-BamHI-Fragment in Antisense-Orientierung in den pBinAR Vektor kloniert (Abb. 2). Das Fragment E enthält das Polyadenylierungssignal des Gens 3 der T-DNA des Ti-Plasmids pTiACH5 (Gielen et al. EMBO J. 3 (1984), 835), Nukleotide 11749-11939, welches als PvuII-HindIII Fragment aus dem Plasmid pAGV 40 (Herrera-Estrella et al. Nature 303 (1983), 209) isoliert worden ist und nach Addition von SphI-Linkern an die PvuII-Schnittstelle zwischen die SphI-HindIII Schnittstelle des Vektors kloniert worden war. Das erhaltene Plasmid BinAR-FNR-Antisense wurde mit Hilfe des Agrobacteriumsystems in Tabak transformiert.

### Beispiel 2

### Pflanzenscreening

Aus den transformierten Tabakpflanzen wurden je 80 Pflanzen regeneriert und ins Gewächshaus transferiert. Die Aktivität der FNR wurde sowohl nach Reinigung der Chloroplasten über direkte Messung der Enzymaktivität als auch über Western-Blot mittels immunochemische Detektion mit dem Biotin-Streptavidin-System der Firma Amersham-Buchler nachgewiesen. Zusätzlich wurde die Expression des Antisense-Gens mit Hilfe von Northern-Blot überprüft.

### Beispiel 3

### Messung der FNR-Enzymaktivität

Die Enzymaktivität wurde nach literaturbekannten Methoden mit isolierten Chloroplasten durchgeführt (Meth. Enzymol. 69 (1980), 250-251).

Die Isolierung von Chloroplasten aus Blättern, die Bestimmung des Frisch- und Trockengewichts sowie des Chlorophyll- und Proteingehalts erfolgte nach Standardmethoden.

### Beispiel 4

### Eigenschaften der Antisense-Pflanze FNR-44

| | Wildtyp | FNR-44 |
|---|---|---|
| Frischgewicht Blätter (g) | 11,3 +/- 1 | 3,2 +/- 0,4 |
| Trockengewicht Blätter (g) | 0,9 +/- 0,1 | 0,2 +/- 0,03 |
| Frischgewicht Wurzeln (g) | 2,7 +/- 0,4 | 0,47 +/- 0,1 |
| Trockengewicht Wurzeln (g) | 0,17 +/- 0,06 | 0,035 +/- 0,01 |
| mRNA (PSL/mm²) | 118 +/- 6 | 15,5 +/- 4 |
| FNR-Aktivität (AU/min*mg) | 4,4 +/- 1,4 | 1,5 +/- 0,6 |

Die Pflanze FNR-44 weist einen geringeren Gehalt an mRNA der FNR und eine reduzierte Enzymaktivität auf. Damit verbunden ist ein reduziertes Wachstum.

Ein Hemmstoff des Enzyms sollte somit ebenfalls das Wachstum von Pflanzen hemmen.

### Beispiel 5

### Testsystem zur Suche nach Hemmstoffen der Ferredoxin:NADP Reduktase.

Neben der physiologisch relevanten reversiblen Reaktion von NADP mit Ferredoxin (Ferredoxinreduktase-Aktivität) katalysiert die Ferredoxin:NADP Reduktase auch die NADPH-abhängige Reduktion von Kaliumhexacyanoferrat-III (Diaphorase-Aktivität). In der Literatur sind Methoden zur Messung dieser Reaktionen beschrieben (Meth. Enzymol., Bände 23 und 69).

Die Diaphorase-Aktivität kann bestimmt werden indem man z.B. Chloroplasten oder isolierte Ferredoxin:NADP Reduktase (Fa. Sigma) in 100 mM Tris/HCl-Puffer, pH 8,2 + 0,5 mM Kaliumhexacyanoferrat-III + 0,5 mM NADPH bzw. einem NADPH-regenerierendem System wie z. B. 0,5 mM NADP + 2,5 mM Glucose-6-phosphat + 1 Unit/ml Glucose-6-phosphat Dehydrogenase bei 25°C inkubiert und die Abnahme der Absorption bei 420 nm verfolgt. Als Blindwert kann ein Inkubationsansatz ohne Ferredoxin:NADP Reduktase verwendet werden.

Die Ferredoxinreduktase-Aktivität kann bestimmt werden indem man z.B. Chloroplastenextrakte oder isolierte Ferredoxin:NADP Reduktase (Fa. Sigma) in 100 mM Tris/HCl-Puffer, pH 7,8 + 0,2 mM NADPH + 0,65 mg/ml Cytochrom c + 0,1 mg/ml Ferredoxin bei 25°C inkubiert und die Absorptionsänderung der 550 nm bzw. die Differenz der Absorptionsänderungen bei 550 nm und 540 nm mißt. Als Blindwert kann ein Inkubationsansatz ohne Ferredoxin verwendet werden.

Die Wirkung einer Testsubstanz kann untersucht werden, indem die Substanz in einem geeigneten Lösemittel wie z. B. Dimethylsulfoxid gelöst wird und ein Aliquot der Lösung zu dem beschriebenen Inkubationsansatz pipettiert wird. Als Kontrolle kann ein entsprechendes Aliquot des reinen Lösemittels verwendet werden. Die Wirkung der Testsubstanz kann durch den Vergleich der Absorptionsänderungen der jeweiligen Inkubationsansätze bestimmt werden.

### Beispiel 6

| Hemmung der Diaphorasereaktion: | | |
|---|---|---|
| | AU/min *10⁻³ | % Hemmung |
| Blindwert¹ | 9,4 | 100 |
| Kontrolle (DMSO) | 31,3 | 0 |
| 50 µM Verbindung I B.3 | 9,0 +/- 1,4 | 102 |

| | | |
|---|---|---|
| ¹ ohne Ferredoxin:NADP Reduktase ² Mittelwert +/- Standardabweichung aus 6 Versuchen | | |

| Hemmung der Ferredoxinreduktasereaktion: | | |
|---|---|---|
| | E (550 -540) | % Hemmung |
| Blindwert¹ | 0.027 | 100 |
| Kontrolle (DMSO) | 0.692 | 0 |
| 1 mM Verbindung I B.3 | 0.093 +/- 0.026 | 90 |

| | | |
|---|---|---|
| ¹ ohne Ferredoxin ² Mittelwert +/- Standardabweichung aus 6 Versuchen | | |

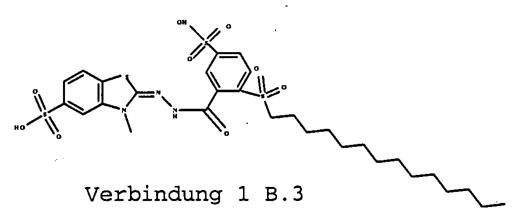

### Beispiel 7

### Hemmstoffe der Ferredoxin-NADP⁺-Oxidoreduktase

### Anwendungsbeispiele

Der Einfluß der erfindungsgemäßen herbiziden Verbindungen auf das Wachstum der Wasserlinse geht aus den folgenden Testergebnissen hervor:

Die Anzucht der Wasserlinse *Lemna paucicostata* erfolgte unter sterilen Bedingungen in 250 ml Glasgefäßen mit 100 ml anorganischer Nährlösung und Zugabe von 1 % Saccharose, wie bei Grossmann et al., Pesticide Science 35( 1992), 283 - 289 beschrieben. Zu Beginn des Tests wurden 150µl einer acetonischen Wirkstofflösung (Stammlösung: 100fach konzentriert) in Petrischalen (Durchmesser 6 cm, Höhe 1,5 cm; Fa. Greiner, Frickenhausen) mit 15 ml Nährlösung (ohne Saccharosezusatz) pipettiert. Den Kontrolltests wurde nur der Lösungsmittelanteil der Wirkstofflösung zur Nährlösung zugegeben. Anschließend wurden 4 *Lemna* Pflanzen pro Schale eingesetzt, die Schalen mit Deckeln abgedeckt und unter Dauerlicht-Bedingungen (Phillips TL white neontubes, ca. 40 µmol m⁻²s⁻¹; λ = 400 - 750 nm) im Klimaschrank bei 25°C inkubiert. Nach 8 - 10 Tagen wurde mit einem Bildanalysegerät (Fa. Imago, Compulog Computer Syst., Böblingen) die Zunahme der Blattfläche als Wachstumsparameter bestimmt und daraus die Wachstumshemmung in % zur Kontrolle berechnet.

Die Ergebnisse der Tests sind der folgenden Tabelle zu entnehmen. Die herbizide Wirkung der erfindungsgemäßen Verbindungen zeigt sich in der starken Hemmung des Wachstums von *Lemna.*

**Tabelle**

| Erfindungsgemäße Verbindungen I | Eingesetzte Konzentration | Wachstumshemmung (% zur Kontrolle) |
|---|---|---|
| A.28 | 10⁻⁴ M | 60 |
| | 10⁻⁵ M | 42 |
| | 10⁻⁶ M | 26 |
| A.46 | 10⁻⁴ M | 56 |
| | 10⁻⁵ M | 33 |
| | 10⁻⁶ M | 13 |
| A.47 | 10⁻⁴ M | 18 |
| A.48 | 10⁻⁴ M | 42 |
| | 10⁻⁵ M | 34 |
| | 10⁻⁶ M | 28 |
| A.49 | 10⁻⁴ M | 77 |
| | 10⁻⁵ M | 49 |
| | 10⁻⁶ M | 19 |
| A.51 | 10⁻⁴ M | 29 |
| A.52 | 10⁻⁴ M | 89 |
| | 10⁻⁵ M | 12 |
| A.55 | 10⁻⁴ M | 28 |
| B.3 | 10⁻⁴ M | 96 |
| | 10⁻⁵ M | 66 |
| | 10⁻⁶ M | 54 |

### Beispiel 8

### Synthese von 3-Methyl-6-n-propylaminosulfonyl-benzthiazolon-2-((2-n-butylsulfonyl)-benzoesäure)hydrazon

0.33 g (0,0013 mol) 2-n-Butylsulfonyl)benzoesäurehydrazid und 0,56 g (0,00126 mol) 2-Methylmercapto-3-methyl-6-(n-propylaminosulfonyl)benzimidazolium-p-toluolsulfat werden in einem Gemisch aus 7 ml Pyridin und 7 ml Piperidin 6 h unter Rückfluß erhitzt. Anschließend wird eingeengt und der verbleibende Rückstand mit Essigester verrührt. Der unlösliche Rückstand wird verworfen, das Filtrat eingeengt und an einer Kieselgelsäure chromatographiert (Methylenchlorid/Methanol 9:1).
Ausbeute: 110 mg (Fp. 125-127°C).

Analog: 3-Methyl-6-sulfonsäurebenzthiazolon-2-((4-n-butylaminosulfonyl)-benzoesäure)hydrazon (Fp. > 250°C).

### Beispiel 9

### Synthese von 3-Methyl-5-sulfonsäure-benzthialzolon-2-((2-n-butylaminosulfonyl)-benzoesäure)hydrazon

0,47 g (0,00172 mol)2-(n-Butylaminosulfonyl)benzoesäurehydrazid und 0,75 g (0,00172 mol) 2-Methylmercapto-3-Methyl-5-sulfonsäurebenzimidazolium-p-toluolsulfat werden in einem Gemisch aus 10 ml Pyridin und 10 ml Piperidin 6 h refluxiert. Anschließend wird mit Wasser verdünnt und mit Salzsäure angesäuert. Der erhaltene Niederschlag wird abgesaugt und getrocknet.
Ausbeute: 280 mg (Fp. 247°C Zersetzung).

Analog: 3-Methyl-5-sulfonsäure-benzthiazolon-2-((4-n-butylsulfonyl)-benzoesäure)hydrazon (Fp. > 250°C).

## Patentansprüche

1. Verwendung des Enzyms Ferredoxin:NADP Reduktase zum Auffinden von Inhibitoren pflanzlicher Ferredoxin:NADP Reduktase mit herbizider Wirkung.

2. Verfahren zur Identifizierung von Stoffen, welche eine potentielle herbizide oder wachstumsregulatorische Wirkung besitzen, die durch eine Hemmung oder Inaktivierung einer pflanzlichen Ferredoxin:NADP Reduktase zustande kommt, **dadurch gekennzeichnet, daß**
a) zunächst das Enzym Ferredoxin:NADP Reduktase durch heterologe Expression einer DNA-Sequenz, die für dieses Transportprotein kodiert, in einem transgenen Organismus oder transgenen Zellen hergestellt wird, anschließend
b) dieser rekombinate Organismus als Ganzes oder ein Zellaufschluß dieses Organismus zur Untersuchung einer chemischen Verbindung auf ihre inhibitorische Wirkung gegen das Enzym Ferredoxin:NADP Reduktase eingesetzt wird und
c) die gegen das Enzym Ferredoxin:NADP Reduktase aktive Verbindung auf ihre herbizide oder wachstumsregulatorische Aktivität gegen Pflanzen geprüft wird.

3. Verfahren gemäß Anspruch 2 **dadurch gekennzeichnet, daß** der transgene Organismus ein einzelliger Organismus oder eine pflanzliche Zelle ist.

4. Verbindungen mit herbizider und/oder wachstumsregulatorischer Aktivität, identifizierbar über Verfahren gemäß den Ansprüchen 2 und 3.

5. Verwendung von Herbiziden und wachstumsregulatorischen Wirkstoffen gemäß Anspruch 4 als Pflanzenschutzmittel, in Verbindung mit anderen Herbiziden, Insektiziden, Fungiziden, Akariziden, Nematiziden und in der Landwirtschaft üblichen Hilfsstoffen.
